# EUROPÄISCHE PATENTANMELDUNG

(11) **EP 1 471 044 A1**
(43) Veröffentlichungstag der Anmeldung: **27.10.2004**
(21) Anmeldenummer: 04100874.9
(22) Anmeldetag: 04.03.2004
(51) Int. Cl.: C07C 13/263

(54) **6-(2,7-Octadienyl)-1,4-Cyclooctadien**

(30) Priorität: 25.04.2003 DE 10318773
(71) Anmelder: Degussa AG, 40474 Düsseldorf (DE)
(72) Erfinder: Wilczok, Norbert, 45481 Mülheim (DE); May, Matthias, 46282 Dorsten (DE); Knaack, Martin, 48249 Dülmen (DE)

(57) **Zusammenfassung**

Die Erfindung betrifft den mehrfach ungesättigten C₁₆-Kohlenwasserstoff 6-(2,7-Octadienyl)-1,4-Cyclooctadien, ein Verfahren zu seiner Herstellung aus Butadien-1,3 an einem Katalysatorsystem bestehend aus einer Nickelkomplexverbindung, einem organischen Phosphit und Dialkylaluminiumalkoxid, und dessen Verwendung als Einsatzprodukt für die Herstellung von Riechstoffen und pharmazeutischen Produkten oder als Vernetzer bei der Herstellung von Gummi.

## Beschreibung

Die Erfmdung betrifft den mehrfach ungesättigten C₁₆-Kohlenwasserstoff 6-(2,7-Octadienyl)-1,4-Cyclooctadien, dessen Herstellung aus Butadien-1,3 und dessen Verwendung. Potentielle Anwendungsgebiete für diesen hochsiedenden C₁₆-Kohlenwasserstoff liegen in der Parfümund Riechstoffindustrie, beispielsweise in der Umsetzung zu makrocyclischen Ketonen (Takasago Perfumery Co., JP 57 021 254, zitiert nach CAN 97: 162 457) oder auch als Einsatzprodukt für die Herstellung von pharmazeutischen Produkten. Eine der wichtigsten Verwendungsmöglichkeiten ergibt sich jedoch als Vemetzer in synthetischen Kautschuken bei der Gummiherstellung zum Beispiel für Reifen oder als Copolymer bei der Herstellung von Kunststoffen wie zum Beispiel von Polyolefinen.

Ausgehend von Butadien-1,3 lassen sich monomere Kohlenwasserstoffe mit einer geraden Anzahl von Kohlenstoffatomen herstellen. Typische Monomere sind dabei Verbindungen mit 12 bis 24 Kohlenstoffatomen, bestehend insbesondere aus einem Ringgerüst mit 6 bis 16 Kohlenstoffatomen und gegebenenfalls einer oder mehreren Seitenketten.

Als wichtige, nicht limitierte Vertreter für solche Monomere seien 3-(2-Butenyl)-1,5,9-cyclododecatrien [34057-87-9], 3-(3-Butenyl)-1,5,9-cyclododecatrien [34057-86-8] und 3-(1-Methylpropenyl)-1,5,9-cyclododecatrien [34057-85-7] sowie Cyclohexadeca-1,5,9,13-tetraen [23579-21-7] genannt.

Während mehrere Verfahren zur Synthese von Cyclohexadeca-1,5,9,13-tetraen ausgehend von Butadien an Nickelkatalysatoren (GB 1 287 252, Toray Industries; DE 19 06 361, Toyo Rayon Co.) oder durch ringöffnende Metathese aus 1,5-Cyclododecadien an Wolfram- (E. A. Ofstead, Macromol. Synth. 1977, 6, 69; US 3 439 057, Goodyear Tire & Rubber Co.), Rhodium- (K. Sato et al., Bull. Soc. Chem. Jpn. 1979, 52, 3192) oder Rhenium-Katalysatoren (US 3 865 888, Goodyear Tire) bekannt sind, sind die anderen Monomerkomponenten bisher kaum in der Literatur beschrieben. Lediglich Mitsubishi Petrochemicals Co. Ltd. hat die Herstellung solcher Monomere an Ziegler-Katalysatoren ausgehend von einem Gemisch aus 1,3-Butadien und Dimeren wie beispielsweise 1,3,7-n-Octatrien beschrieben. Patentdokumente hierzu sind DE 20 63 348, US 3 658 926 und JP 48 019 304 (zitiert nach CA: 79:78229).

Insbesondere werden als Verbindungen neben dem Cyclododecatrien C₁₆-Kohlenwasserstoffe beschrieben, die eine C₁₂-Ringstruktur aufweisen.

Es bestand die Aufgabe, weitere cyclische, insbesondere relativ kleingliedrige Kohlenwasserstoffe mit Eigenschaftsbildern herzustellen, die die bekannten in vorteilhafter Weise abwandeln und ergänzen.

Es wurde nun überraschend gefunden, dass bei der Synthese von Cyclooctadien aus Butadien-1,3 6-(2,7-Octadienyl)-1,4-Cyclooctadien, ein bisher nicht bekannter C₁₆-Kohlenwasserstoff mit einem C₈-Ring, hergestellt werden kann, welcher sich als Einsatzkomponente zum Beispiel bei der Herstellung von Riechstoffen, als Vemetzer zum Beispiel im Kautschukbereich oder als Copolymer zum Beispiel in Polyolefinen eignet. Die Synthese gelingt insbesondere mit einem Katalysatorsystem, das aus einer Nickelkomplexverbindung, einem organischen Phosphit und einem aktivierenden Dialkylaluminiumalkoxid besteht.

Als Nickelverbindungen eignen sich Verbindungen, die durch die Aktivierung (Reduzierung) Nickel(0)-Verbindungen ergeben. In erster Linie gehört dazu das Nickelacetylacetonat.

Die Phosphite enthalten vorzugsweise aromatische Gruppen. Besonders geeignet sind Tris-(o-Phenylphenyl)-phosphit und/oder Tris-(2,4-di-tert.butylphenyl)-phosphit .

Die aktivierende Dialkylaluminiumkomponente enthält als Alkylgruppen Reste mit 1 bis 4, vorzugsweise 2 Kohlenstoffatomen. Es seien als Beispiele der Methyl-, insbesondere aber der Ethyl-, der n- und iso-Propyl- und die Butyl-Reste in ihren verschiedenen isomeren Formen genannt, wobei auch gemischte Dialkylverbindungen verwendet werden können. Der Alkoxidrest enthält ebenfalls 1 bis 4, bevorzugt 2 Kohlenstoffatome, wobei die vorgenannte Aufzählung sinngemäß gilt. Als besonders bevorzugtes Dialkylaluminiumalkoxid sei das Diethylaluminiumethoxid genannt.

Nach der Umsetzung von dem Butadien-1,3 und der destillativen Abtrennung von eventuell nicht-umgesetztem Butadien-1,3 und Cyclooctadien in einer Vakuum-Stufe (Flash-Verdampfung) verbleibt im Sumpf ein Produktgemisch, das C₁₂- , C₁₆- und C₂₀-Kohlenwasserstoffe neben weiteren Hochsiedem enthält, aus dem in einer weiteren Vakuumdestillation C₁₂- von C₁₆-Verbindungen getrennt und 6-(2,7-Octadienyl)-1,4-Cyclooctadien (Sdp. 127 °C bei 3 mbar) isoliert werden kann, welches überraschend stabil ist.

### Beispiel

Das folgende Beispiel soll die Erfindungsgegenstände Stoff und Verfahren zu seiner Herstellung erläutern, ohne auf die speziell genannten Umstände einzuschränken.

### Herstellung der Reinkomponente

Es erfolgte zunächst die Umsetzung von Butadien-1,3 in Gegenwart von Nickelacetylacetonat, Tris-(o-Phenylphenyl)-phosphit und Diethylaluminiumethoxid. Nach destillativer Abtrennung unumgesetzten Butadiens, entstandenen Cyclooctadiens und Cyclododecatriens erfolgte die Isolierung des 6-(2,7-Octadienyl)-1,4-Cyclooctadiens im Vakuum bei 2 - 6 mbar und 125 - 135 °C.

### Analyse auf Reinheit

Die Analyse des Stoffes auf Reinheit wurde an einem Gaschromatographen mit Flammenionisationsdetektor Supelcowax TM 10, Länge 30 Meter, bestimmt.

Das Produkt ist bei 20 °C (Raumtemperatur) flüssig, wasserklar und riecht praktisch nicht.

### Stoffcharakterisierung

Die Identifizierung und Strukturaufklärung erfolgte mit Hilfe von ein- und zweidimensionaler homo- und heteronuklearen NMR-Spektren (H-1 und C-13-NMR-Spektren) sowie Infrarot-Spektren als C₁₆H₂₄- Kohlenwasserstoff 6-(2,7-Octadienyl)-1,4-cyclooctadien folgender Struktur:

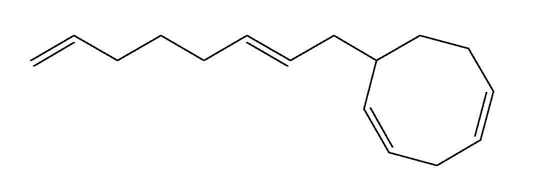

Dabei wurde die Struktur NMR-spektroskopisch durch H,H-COSY-, HMQC-, HMBC-, 1,1-ADEQUATE- und insbesondere durch INADEQUATE-Experimente charakterisiert. Die cis-Anordnung der beiden Doppelbindungen im Ring wurde durch ein H-1-NMR-Experiment mit selektiver Entkopplung untermauert. Die Existenz einer trans-Doppelbindung wurde IRspektroskopisch nachgewiesen.

## Patentansprüche

1. 6-(2,7-Octadienyl)-1,4-Cyclooctadien.

2. Verfahren zur Herstellung von 6-(2,7-Octadienyl)-1,4-Cyclooctadien aus Butadien-1,3,
**dadurch gekennzeichnet, dass**
Butadien-1,3 an einem Katalysatorsystem bestehend aus einer Nickelkomplexverbindung, einem organischen Phosphit und Dialkylaluminiumalkoxid umgesetzt wird, restliches Butadien-1,3, entstandenes Cyclooctadien und Cyclododecatrien abdestilliert werden und aus dem Rückstand 6-(2,7-Octadienyl)-1,4-Cyclooctadien im Vakuum fraktioniert destilliert wird.

3. Verfahren nach Anspruch 2,
**dadurch gekennzeichnet, dass**
als Nickelkomplexverbindung Nickelacetylacetonat verwendet wird.

4. Verfahren nach Anspruch 2,
**dadurch gekennzeichnet, dass**
als Phosphit Tris-(o-Phenylphenyl)-phosphit und/oder Tris-(2,4-di-tert.butylphenyl)-phosphit verwendet wird.

5. Verfahren nach Anspruch 2,
**dadurch gekennzeichnet, dass**
als Dialkylaluminiumalkoxid Diethylaluminiumethoxid verwendet wird.

6. Verfahren nach Anspruch 2,
**dadurch gekennzeichnet, dass**
das 6-(2,7-Octadienyl)-1,4-Cyclooctadien durch Vakuumdestillation gewonnen wird.

7. Verwendung des 6-(2,7-Octadienyl)-1,4-Cyclooctadiens als Einsatzprodukt für die Herstellung von Riechstoffen und pharmazeutischen Produkten.

8. Verwendung des 6-(2,7-Octadienyl)-1,4-Cyclooctadiens als Vernetzer bei der Gummiherstellung oder als Copolymer bei der Herstellung von Kunststoffen.
